# EUROPEAN PATENT SPECIFICATION

(11) **EP 0 670 726 B1**
(45) Date of publication and mention of the grant of the patent: **20.03.2002**
(21) Application number: 94929871.5
(22) Date of filing: 23.09.1994
(51) Int. Cl.: A61K 35/10, A61K 39/39

(54) **TREATMENT OF HIV INFECTION WITH HUMIC ACID**
BEHANDLUNG VON HIV-INFEKTIONEN MITTELS HUMINSÄURE
TRAITEMENT D'UNE INFECTION HIV A L'AIDE D'ACIDE HUMIQUE

(30) Priority: 24.09.1993 US 126312; 22.09.1994 US 310675
(43) Date of publication of application: 13.09.1995
(73) Proprietor: Zanetti, Maurizio, La Jolla, CA 92037 (US)
(72) Inventor: Zanetti, Maurizio, La Jolla, CA 92037 (US)
(74) Representative: Armitage, Ian Michael
(86) International application number: US9410775
(87) International publication number: WO9508335

(56) References cited:
- EP-A- 0 537 430
- EP-A- 0 540 945
- WO-A-90/04412
- WO-A-92/16216
- WO-A-94/18957
- 8TH. INTERNATIONAL CONFERENCE ON AIDS / 3RD. STD WORLD CONGRESS, AMSTERDAM, NL, 19-24 JULY, 1992, AMSTERDAM page 36 J. SCHNEIDER ET AL. 'INHIBITION OF HIV-1 INFECTION BY SYNTHETIC HUMINATES.'
- PATHOLOGY, RESEARCH AND PRACTICE (78TH. MEETING OF THE GERMAN SOCIETY OF PATHOLOGY, 5-9 APRIL 1994, ZÜRICH, CH), vol.190, no.3, March 1994, STUTTGART, DE page 245 J. SCHNEIDER ET AL. 'HIV-VIROSTATIC EFFECTS OF HUMIC ACIDS.'

## Description

### Field of the Invention

The present invention is based upon the finding of anti-human immunodeficiency virus activity of a natural and commercially available material known as humic acid. Humic acid is a dark color organic material which can be extracted from soil by various reagents and which is insoluble in dilute acid.

### Background of the Invention

Acquired immunodeficiency syndrome (AIDS) is a complex disease caused by the human immunodeficiency virus (HIV). There are an estimated 12.9 million infected people world-wide. Of these about one-fifth have developed AIDS and have died. The disease represents an incredible threat to society both in industrialized and developing countries.

Measures to control HIV infection are still limited and are essentially based on 1) avoidance by safe sex procedures, 2) development of a vaccine for the containment of HIV infection or the treatment of already infected individuals, and 3) treatment by anti-viral chemotherapy. While the first measure is subject to social, political and religious factors, a vaccine for AIDS might take years before becoming available. To date there are only a few approved treatments for AIDS which in themselves offer very little in terms of long-term relief to patients with this disease.

Unapproved therapies and investigational drugs have become an important area of research and hope for both the patients as well as the scientific and political establishments. Of the three nucleoside analogs that are the corner stone of HIV-1 therapy, Zidovudine (AZT) is the only one that may prolong survival. However, the range of toxicity with limited efficacy and the developing resistance to this and similar drugs (ddc, ddi, IFN, Acylovir, Foscarnet for Acylovir-resistant herpes viruses, hemopoietic growth factors, HIV protease inhibitors, etc.) are matters of major concern. Nucleoside analogs such as didanosine undergo intracellular conversion to active triphosphate metabolite. The active metabolite appears to inhibit viral reverse transcriptase and terminate the transcription of proviral DNA. At clinically relevant concentrations these analogs are expected to produce inhibition of actively replicating HIV-1. However, the effect of didanosine on clinical end-points remains to be established, and peripheral neuropathy and pancreatitis are the dominant adverse effects. Several issues remain unresolved including the precise clinical benefit, pronounced toxicity, induction of allergic reactions, drug-drug interactions complicating the pharmacokinetics and drug intolerance.

The present effort was based on a goal of developing non- or less toxic therapy for AIDS. Herein is reported one such effort that suggests a safe alternative to existing therapeutic strategies. With this invention is demonstrated that Humic Acid, a natural, commercially available product extracted from soil, demonstrates potent anti-HIV activity in vitro. Also shown is the anti-viral activity of Humic Acid associated with an immunostimulatory effect mirrored by an enhanced production of interleukin-2 (IL-2) by human lymphocytes upon activation with mitogens like plant lectins.

Kloecking et al. in Experientia 28(5), 607 (1972) reported on in vitro inhibition of Coxsackie virus A9 by a compound derived from humic acid. Thiel *et al.* in Zbl. Bakt. Hyg., I. Abt. Orig. A 239, 304 (1977) reported on *in vitro* studies on the antiviral activity against Herpes Simplex virus with a certain component obtained from a humic acid source. Hils et al. in Biomed. Biochim. Acta 45(9), 1173 (1986) reported on the inhibitory effects of humic acid derivative compounds on influenza virus type A as assessed in allantoic egg shells. Wagner et al. in Chem. Abstracts 113, 65270j (1990) reported on transdermal treatment for a herpes-virus-induced rash which consists of a mixture of compounds, possibly including humic acid or derivatives thereof. Riede et al. in Chemical Abstracts 118, 247623n (1993) reported on a specific synthetic humate-like compound that apparently showed signs of inhibiting HIV infection and an oral presentation reporting similar findings has been purported to have taken place in Berlin on or about 8 June 1993 by a J. Schneider. Loya, *et al.,* Journal of Natural Products, 56, 2120 (December 1993) also pertains.

### Summary of the Invention

The present invention demonstrates that soil extracted Humic Acid (denoted here in as HA90 or AVC) can be used in the treatment of HIV infection by suppressing viral replication, inhibiting cell fusion between infected and noninfected cells, and boosting T cells to product a soluble factor key to the physiology of the immune system.

AVC also exhibited immunostimulatory effects acting as a costimulant of plant lectins on the activation of human lymphocytes and enhancing the production of IL-2, a genetically unrestricted, soluble factor produced by T lymphocytes following stimulation with mitogens, allogeneic cells, and antigen.

Thus, in a first aspect, the present invention provides the use of the humic acid for the preparation of a medicament for the inhibition of human immunodeficiency virus in leukocytes infected by said virus.

In a further aspect, the use of the humic acid as a co-stimulant for the preparation of a medicament for immunostimulating IL-2.

In a further aspect, the use of humic acid as an adjuvant for the preparation of a medicament for the immunovaccination of a patient against human immunodeficiency virus or acquired immunodeficiency syndrome, or depressed immune function.

Leukocytes are inclusive of peripheral blood mononuclear cells, lymphocytes resident in lymphoid organ and lymphocytes infiltrating tissues other than lymphoid organ. They can be contacted ex vivo or in vivo when appropriately administered.

### Detailed Description of the Invention

### A. Material Used in this Invention

Humic Acid was purchased from Aldrich Chemical Co., and is referred to herein as such as AVC. It is also referred to herein as anti-viral compound (AVC). It has an appearance of black granules, it contains 30.2% residues on ignition and an elemental analysis demonstrating: Carbon 40.2%, Hydrogen 3.5% and Nitrogen 3.5%. In addition it contains elements of iron (1.4%), sodium (7700 ppm µg/g), aluminum (3500 ppm µg/g), and calcium (5500 ppm µg/g).

Humic Acids have been described as allomelanins found in soils, coals and peat, resulting from the decomposition of organic matter, particularly dead plants. It has been described as consisting of a mixture of complex macromolecules having polymeric phenolic structures with the ability to chelate with metals, especially iron. See Nicholaus, Melanins pp. 147-153 (Hermann, Paris 1968). See also Steelnick J. Chem. Ed. 40, 379 (1963). Additional consulting references include Flaig, Soil Components pp. 1-219 (Gieseking Ed., Springer, Berlin 1975) and Humic Substances II Hays *et al.* Ed., Wiley Interscience, John Wiley, New York (1989), as well as Humus Chemistry, Genesis Composition Reactions, author F.J. Stevenson, John Wiley & Sons, New York (1994). The latter text contains considerable information on the chemistry of the components of humus and in particular in the chapter entitled "Reactive Functional Groups" points out that the major elements in humic acid are carbon and oxygen, the carbon content generally ranging from 53.8 to 58.7% and the oxygen content varying from about 32.8 to 38.3%. Percentages of hydrogen, nitrogen and sulfur are considerably lower. Disregarding sulfur, it is stated that the average chemical formula for humic is C₁₀H₁₂O₅N. It also discloses certain structural groups of organic molecules that are considered important. Among those listed are classes of quinones and hydroxyquinones.

### B. Figure Legends

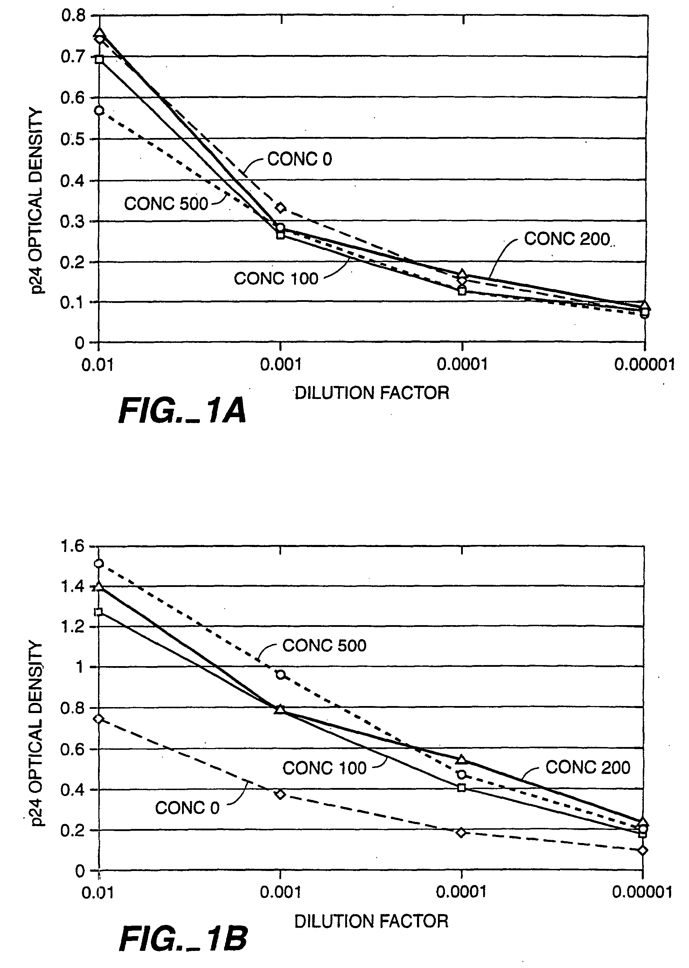

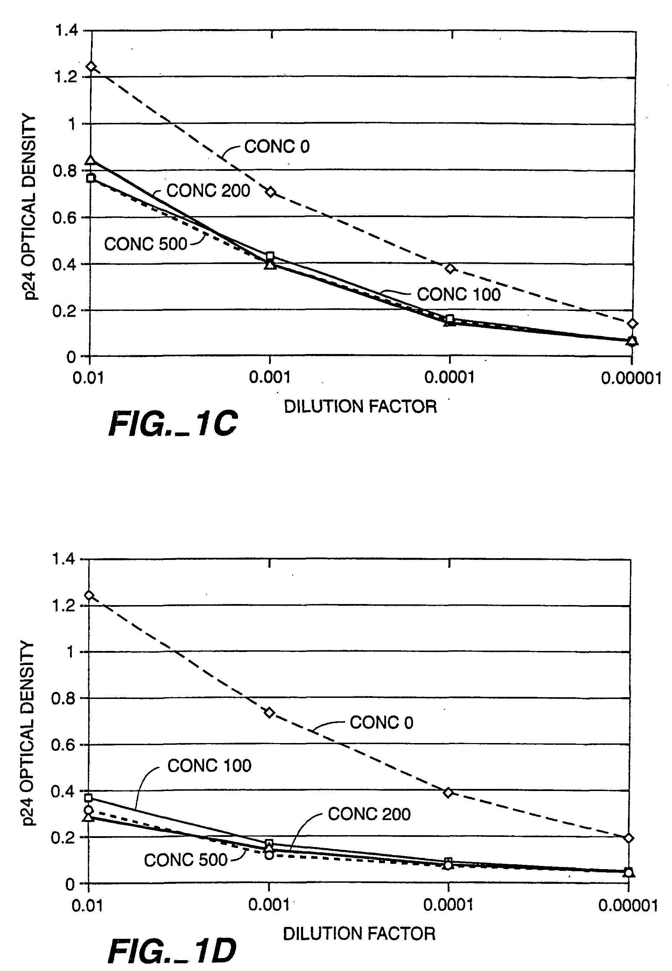

Figure 1. Production of p24 by infected cells in the presence of different concentration of HA.90 at different times. Day O (upper panel); day 2 (second panel from top); day 5 (third panel from top); and day 8 (bottom panel). Serial dilution of the culture supernatant (10⁻¹-10⁻⁴) were used for the assay. p24 was detected in a ELISA capture assay (Abbott kit).

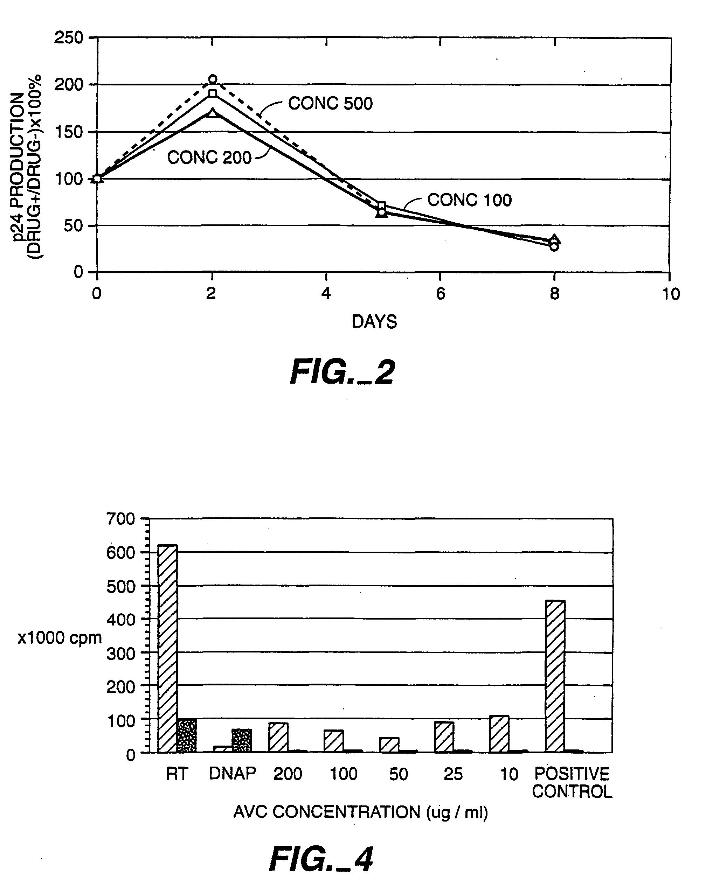

Figure 2. Percent suppression of HIV production in Jurkat cells. The effect of various doses of HA.90 (AVC) on the production of p24 at various times (day 0-8) is represented as percentage p24 production of the amount of p24 produced by control cultures in which Jurkat cells were infected and incubated in medium alone.

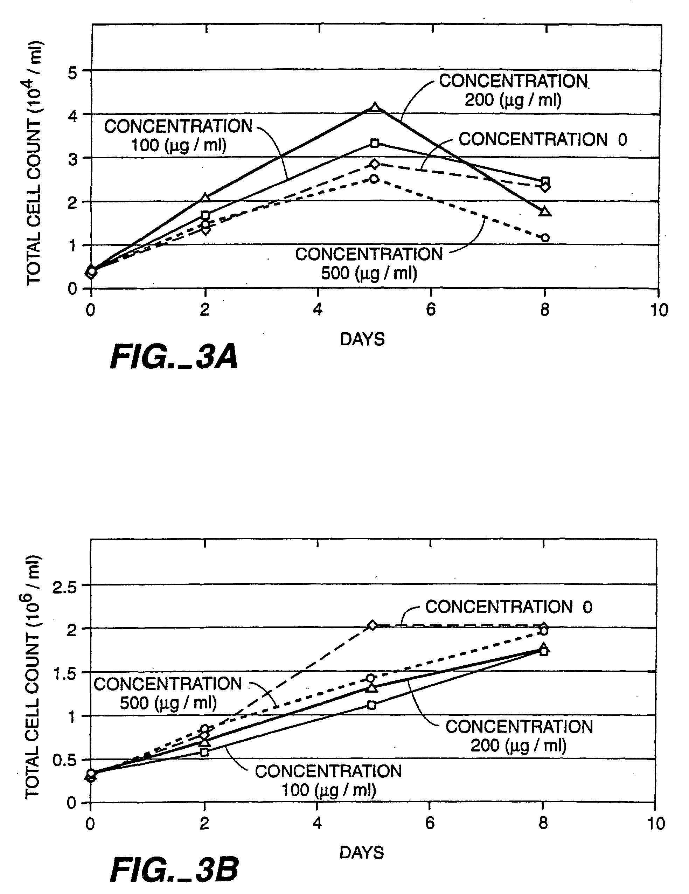

Figure 3. Total cell number during incubation with HA.90 (AVC) at various concentrations. The number of cells during incubation with HA.90 (AVC) is shown for uninfected cells (upper panel) and HIV infected cells (lower panel).

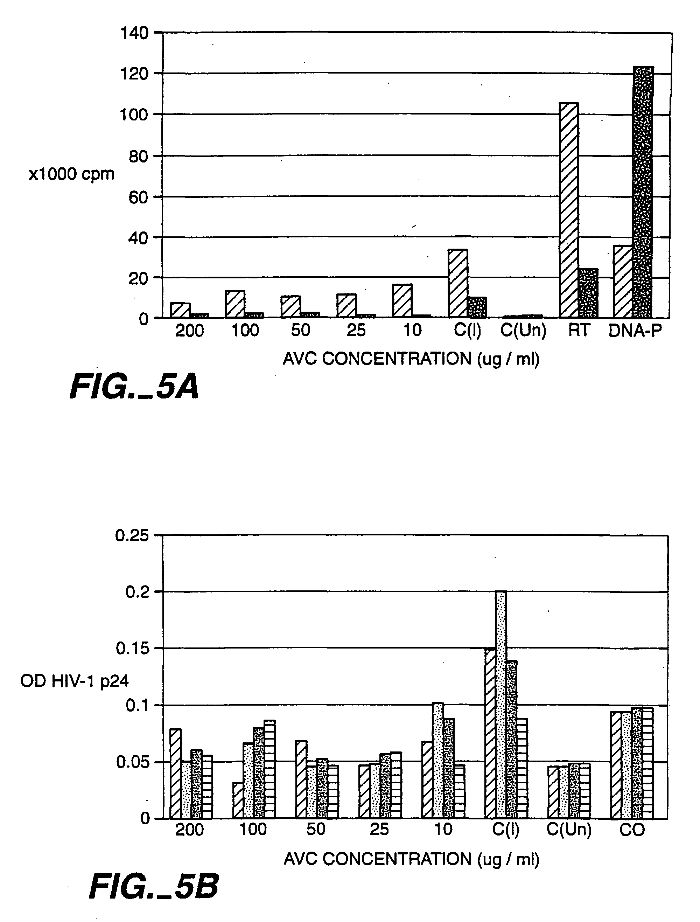

Figure 4. Production of reverse transcriptase in Jurkat cells treated with different concentrations of AVC. Reverse transcriptase was assayed on day 6 using synthetic template-primer sets oligo dtrA and dtdA.

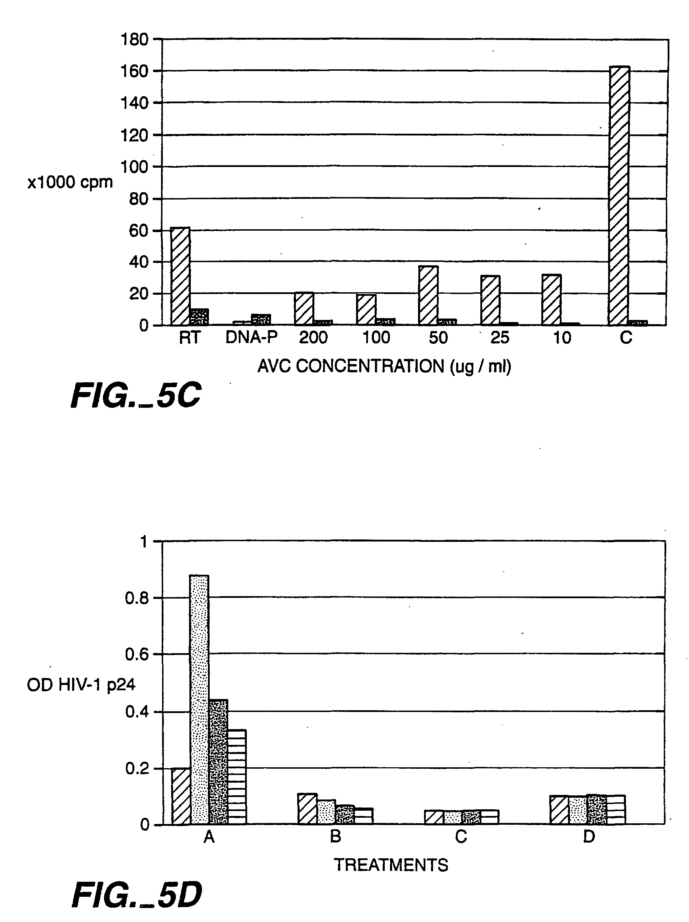

Figure 5. The effect of AVC treatment of HIV-1 replication in PBMC from infected donors and on replication of HIV-1 transmitted in vitro from HIV-1 positive to normal donor PBMC. (A) RT levels after AVC treatment of PBMC from HIV-1 positive donors. These cells were treated with varying concentrations of AVC (10, 25, 50, 100 and 200 µg/ml) and cell culture supernatant was harvested on day 6 and assayed (12). HIV-1 RT and DNA-P were used as positive and negative controls. Symbols: Exogeneous primer-template used dtra (left diagonal lines) and dtdA (solid black). **(B)** HIV-1 p24 determination in day 6 cell culture media from HIV-1 positive donors. HIV-1 p24 determinations were made using Abbott HIVAG-1 kits. The kit cut off (CO) value was less than 0.1 (0.093) OD units. Bars represent harvest of cell culture supernatant on day 6 (right diagonal lines), 9 (dotted), 12 (solid black), and 15 (horizontal lines). C(1) = infected cells, positive control, C(UN) - uninfected cells, negative control. (C) Effect of AVC on RT levels of HIV-1 transmitted from HIV-1 positive donor cells to normal PBMC in a co-culture experiment. Cell culture supernatants were harvested from a culture preadjusted to 1×10⁶ cells/ml 24 before the harvest. These cells were treated with different concentrations of AVC (10, 25, 50, 100, and 200 ug/ml) on day 3. HIV-1 RT and DNA-P were used as positive and negative controls respectively. C= untreated control. Assays were done in the presence of Mg + + as a divalent cation. Symbols: dtrA (left diagonal lines) and dtdA (solid black). C = untreated infected cells. **(D)** Effect of AVC treatment on expression in normal PBMC transmitted with HIV-1 from HIV-1 positive donor cells in a co-culture experiment. Cells were then treated with IL-2 with or without AVC. Media for the HIV-1 p24 assay were harvested on days 6 (right diagonal lines) 9 (dotted) 12 (solid black) and 15 (horizontal lines). A IL-2, B = AVC, C = negative control, and D= kit cut off value below which all specimens are considered nonreactive.

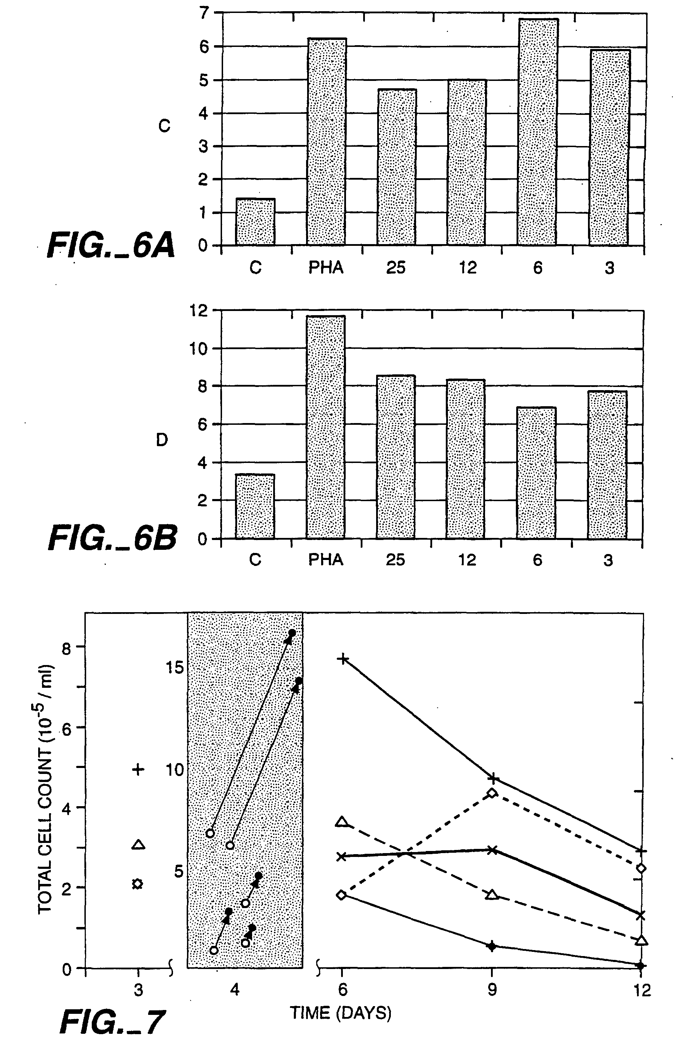

Figure 6. The effect of AVC on cell number and viability. **(C)** Freshly obtained PBMC from normal donors and **(D)** PBMC from HIV-1 infected individuals were isolated from heparinized blood using a ficoll-Hypaque (Histopaque 1077, Sigma) density gradient, washed, and resuspended in RPMI 1640 containing 15% FCS and purified PHA (5 µg/ml) (Burrough-Wellcome, Greenville, N.C.). On day 3 the medium was changed and natural IL-2 (Boheringer-Mannhein) was added (10% v/v). AVC was added on day 3 (3-25 µg/ml). Cells were harvested three days later (day 6) and viability and total cell count assessed by Trypan blue dye exclusion. The experiment shown represents the average of two individuals in each case.

Figure 7. Longitudinal study on cell viability and proliferation of PBMC from HIV-1 infected donors treated with AVC. Data represent average values of three donors tested separately. All cells were activated with PHA (5 µg/ml) at the beginning of culture. Culture conditions were as follows: IL-2 (+); IL-2 plus AVC (Δ); IL-2 added on day 6 (◇); AVC plus IL-2 on day 6 (x); Untreated control (◆). The inset refers to total cell counts on day 4 of PBMC from five HIV-1 infected donors different from those used for the longitudinal survey. All HIV-1 infected donors were under AZT treatment. (○) Baseline cell counts of PHA-activated PBMC; (●) cell counts of PBMC activated with PHA plus AVC.

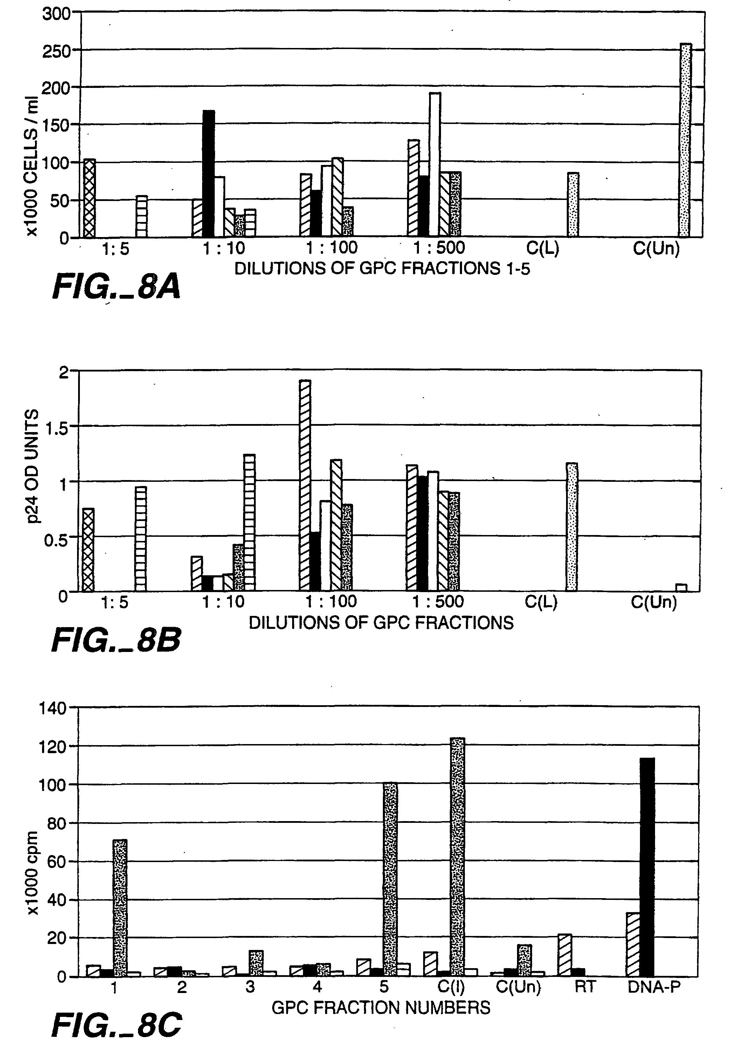

Figure 8. The effect of different fractions of AVC on HIV-1 expression and viability of normal PBMC infected with HIV-1₄₁₀₅. **(A)** Viability of HIV-1 infected PBMC from normal donors after treatment with GPC fractions of AVC (5 ug/ml). Cell viability, and p24 expression (Fig. 4B) were determined in the same cells and culture supernatant. Fractions were diluted (1:5, 1:10, 1:100, and 1:500) and infected cells were treated continuously for 6 days. Symbols: Untreated (cross-hatched) Fraction 1 (right diagonal lines) Fraction 2 (solid black) Fraction 3 (□) Fraction 4 (left diagonal lines) Fraction 5 (dotted) Buffer (horizontal lines) and C(I) and C(UN) □ Buffer was not used in 1:100 and 1:500 dilutions for testing. Buffer was tested by itself and in 1:10 dilution with culture media. C(I) = infected control cells and C(UN) = uninfected control cells. (B) Effect on HIV-1 p24 expression after treatment with GPC fractions of AVC. Cell culture supernatant from HIV-1 infected normal donors were harvested on day 6, viable cell number was adjusted to 1×10⁶ /ml 24 hrs before assay then specimen were taken for p24 assays. HIV-1 p24 levels were determined by the Abbott HIVAG-1 kit. GPC fractions were diluted 1:5, 1:10, 1:100 and 1:500. Buffer was tested alone and included only in 1:10 dilution for testing. Symbols: Untreated cells (cross hatched lines) Fraction 1 (right diagonal lines) Fraction 2 (solid black) Fraction 3 (□) Fraction 4 (left diagonal lines) Fraction 5 (dotted) Buffer ( horizontal lines) and C(I) and C(UN) (□) C(I)= Infected cells and C(UN)= Uninfected cells. (C) Levels of RT expression in cell culture supernatant of HIV-1 infected PBMC from normal donors. Cells obtained from the same normal donors as in Fig 4A and 48 were treated with the same GPC fractions using 1:10 final dilution only and cell culture supernatants were harvested on days 6 and 9. The cell number 24 hrs. before harvest of the supernatant was adjusted to approximately 1×10⁶/ml before each harvest. 1 through 5 are GPC fractions of AVC. C(I) = Infected cells and C(UN) = uninfected cells. Symbols: day 6 dtrA (right diagonal lines) and day 6 dtdA (dotted) day 9 dtrA (solid black) and day 9 dtdA (horizontal lines).

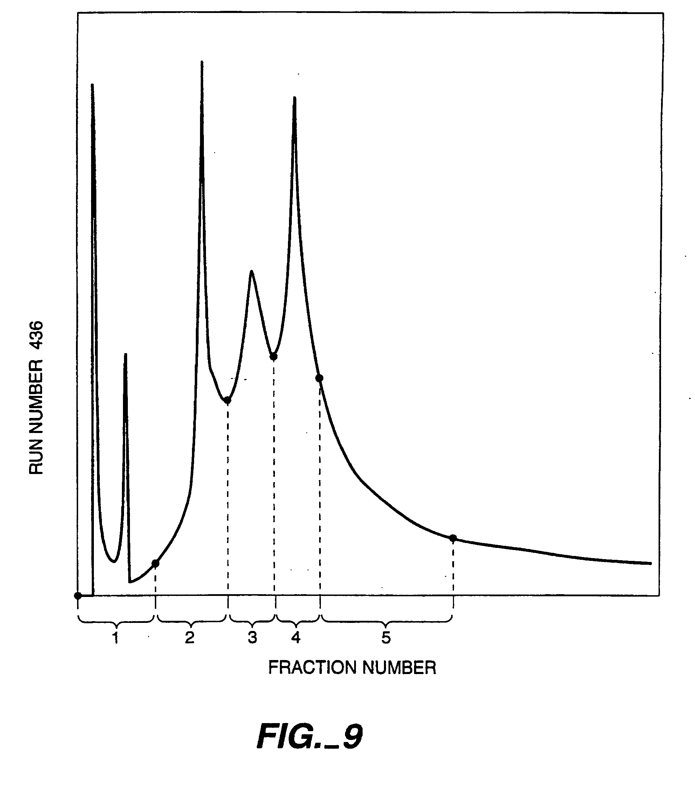

Figure 9. Fractionation of HA.90 by analytical reverse-phase liquid chromatography (RPLC) following a standard protocol. The resulting five fractions consisted of approximately 16, 10, 19, 22 and 26% (w/v), respectively, of the starting material.

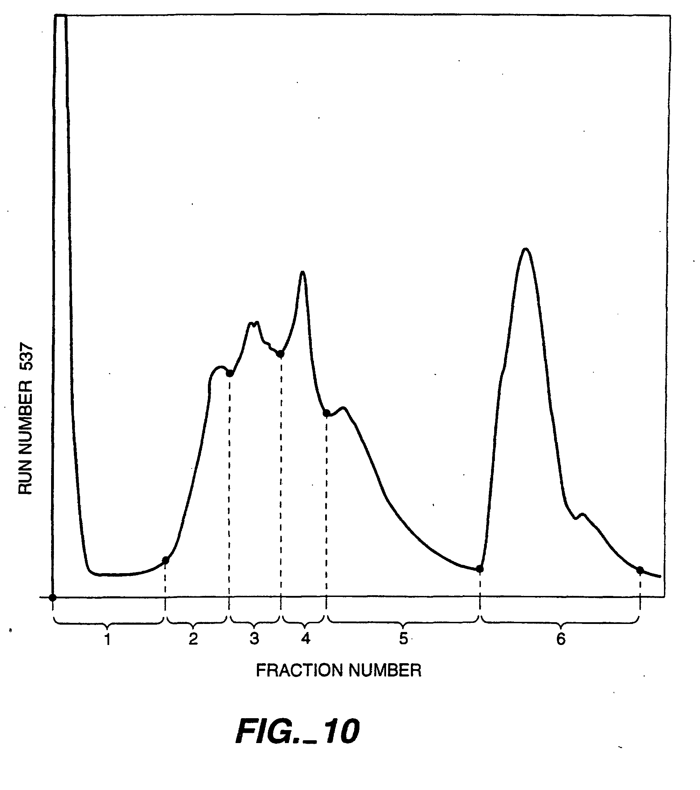

Figure 10. Fractionation of HA.90 by semipreparative reverse-phase liquid chromatography (RPLC) following a standard protocol. The resulting six fractions consisted of approximately 15, 10, 4, 11, 9 and 51% (w/v), respectively, of the starting material.

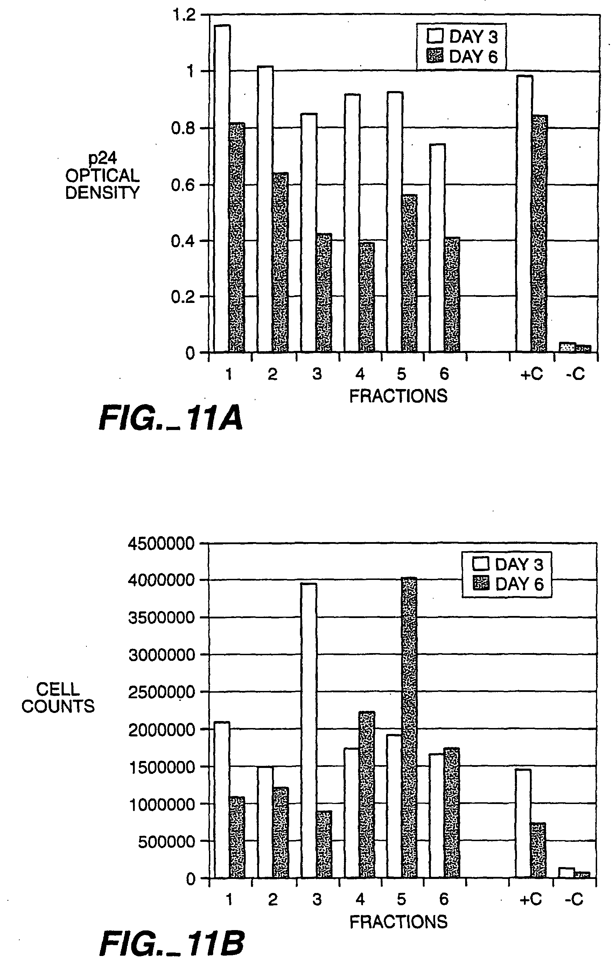

Figure 11. Inhibition of p24 and cell viability of PBMC infected with HIV-1 by RPLC fractions 1 to 6 (see Figure 10).

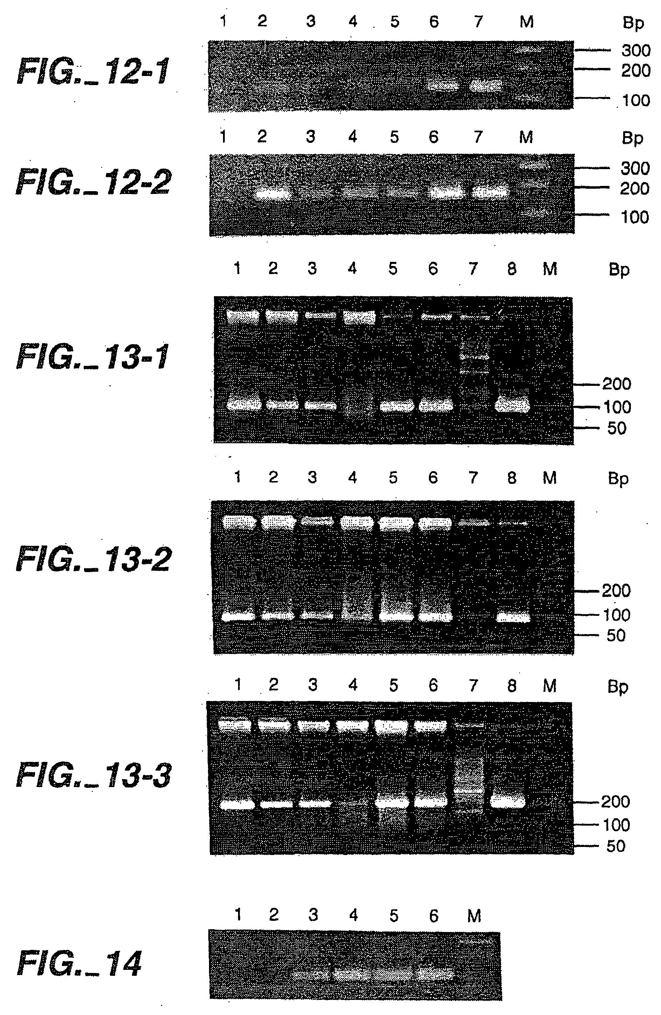

Figure 12. The effect of different fractions 1 to 5 (see Figure 9) of AVC on HIV-1 mRNA in Jurkat cells infected with pNLF-3. Jurkat cells stably transfected with pNLF-4 plasmid were added to uninfected Jurkat cells at a ratio of 1:100. Next day AVC fractions 1 to 5 were added to cells at a concentration of 5µg/ml. Five days later cells were collected, counter, and mRNA was extracted. RT-PCR of mRNA was performed using two pairs of primers discriminating multiply spliced (A1) and unspliced (A2) HIV-1 mRNA. Numbers correspond to fractions 1 to 5 (lane # 2 to 6), lane 7 = mRNA from cells not treated with AVC, lane 1 = negative control.

Figure 13. The effect of different fractions 1 to 5 (see Figure 9) of AVC on HIV-1 proviral DNA in Jurkat cells infected with pNL4-3 (see Figure 12). The effect of AVC fractions on HIV-1 proviral DNA synthesis was tested using primers amplifying portions of DNA synthesized at the beginning (B1), at the intermediate stage (B2) and at the end of HIV-1 DNA synthesis (B3). Fractions 1 to 5 correspond to lanes 1 to 5, lane 6 = DNA isolated from cells not treated with AVC, lane 7 = negative control, lane 8 = positive control, and M - molecular marker. The size of bp is indicated on the right hand margin.

Figure 14. The effect of AVC fractions on polymerase chain reaction analyzed using pNL 4-3 as a template and SK38/39 as set of primers. AVC fraction 4 was diluted with distilled water (1:10, 1:100, 1:200, 1:500, and 1;1000) in lanes 1 to 5. Lane 6 = PCR with distilled water only. The bp size is indicated in right hand margin.

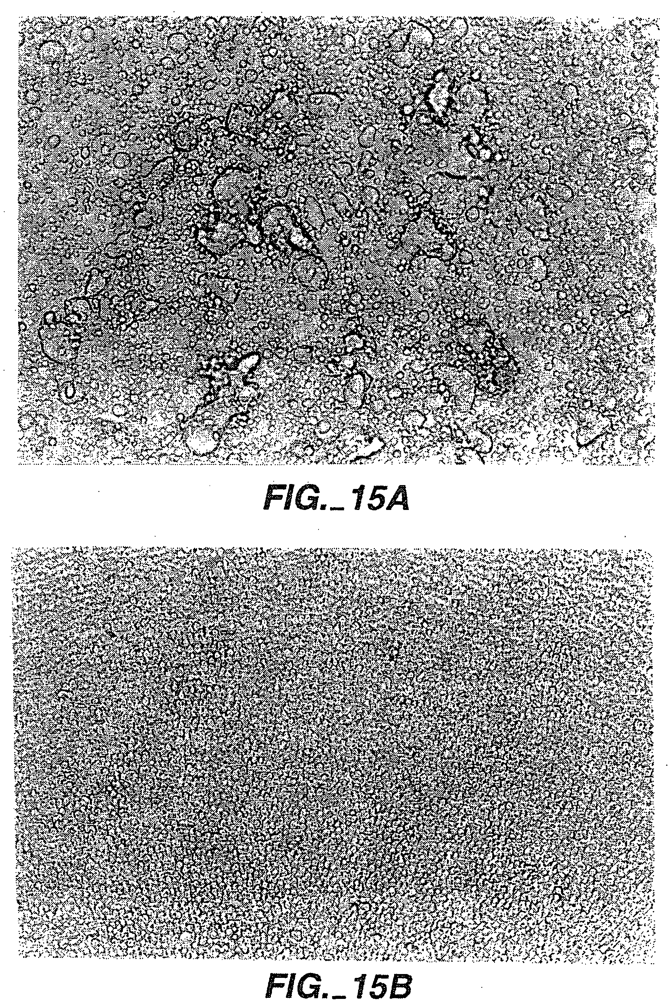

Figure 15. Inhibition of syncytia formation by HA.90. Upper panel shows the formation of syncytia between 8E5 infected cells and noninfected MOLT3 T lymphoma cells. Syncytia are denoted by the large multicellular structures with irregular shape. Lower panel represents the effect of Humic Acid (50 µg/ml) on the formation of syncytia as they are seen in upper panel.

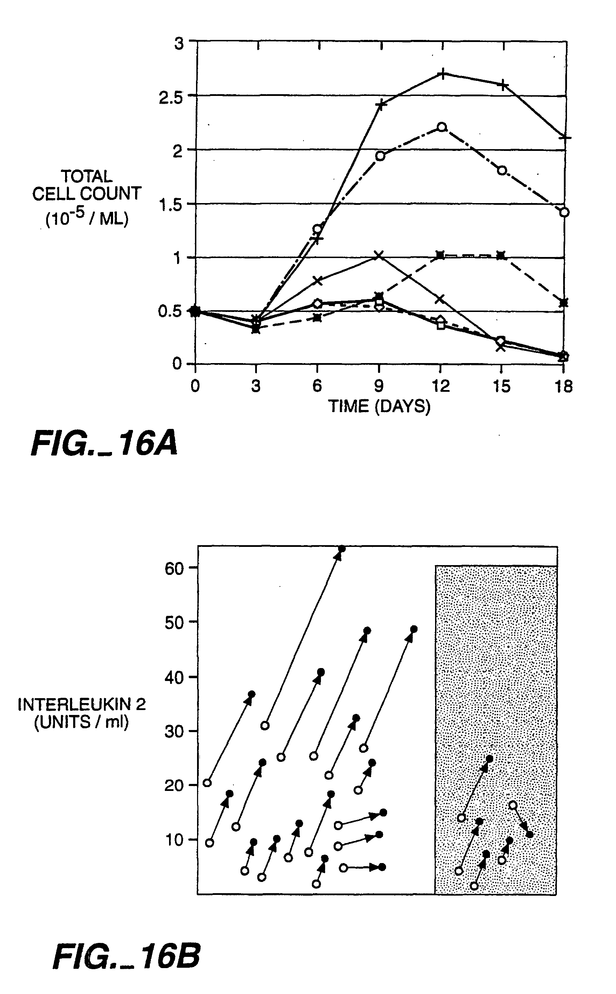

Figure 16. Cytoprotection and potentiation of IL-2 production by AVC in normal PBMC. **(A)** Freshly obtained PBMC were isolated from heparinized blood of healthy donors. On day 3 the culture medium was changed with fresh medium containing natural IL-2 with or without AVC (25 µg/ml). Viable cells were counted manually using Trypan blue dye exclusion every three days. Culture conditions were as follows: IL-2 (+), IL-2 plus AVC (■), AVC **(X)**, AVC treated cells given IL-2 on day 7 (*), untreated control (■), and control culture given IL-2 on day 6 (◆). Results refer to the mean values of three normal donors in three separate experiments. (B) Freshly obtained PBMC from normal donors isolated as detailed above were activated with concanavalin A (Con A) (5µg/l) or PHA (10µg/ml) and cultured as detailed. The IL-2 activity was assayed using the IL-2 dependent murine cell line CTLL-2 as an indicator cell population. These cells constituitively express high levels of IL-2 receptor. The results were quantified by probit analysis (8). A unit of IL-2 activity per ml was defined as the level of IL-2 that yields 50% of maximal stimulation based on a standard rat IL-2 supernatant. CTLL-2 cells were seeded (100 µl, 1 X 10⁴/well) in triplicate 96-well microtiter plates and cultured for 24 hours in RPMI 1640 supplemented with 5% FCS and 2-mercaptoethanol in the absence (background response) or in the presence of serial dilutions of test culture supernatants.
3H-TdR uptake was determined in a four hour terminal pulse (1 µCi/well). (○) Baseline IL-2 production of PHA-activated PBMC; (●) IL-2 production by PBMC activated with PHA plus AVC.

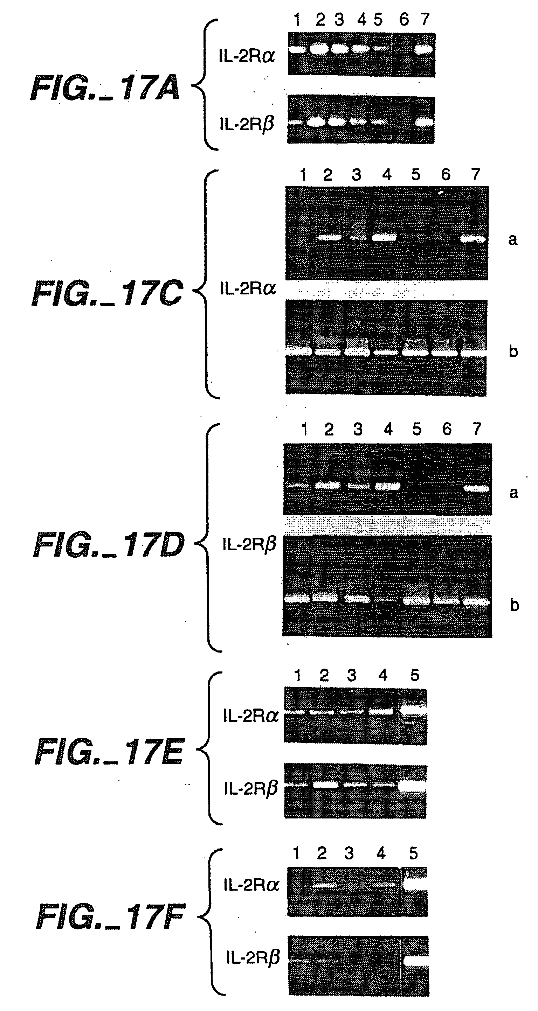

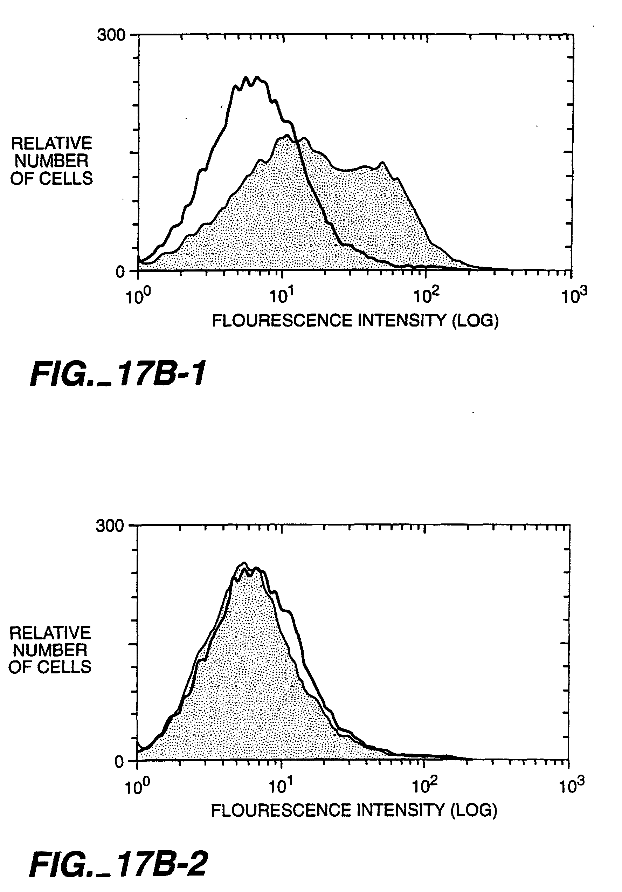

Figure 17. IL-2 receptor expression in uninfected PBL and PBL from HIV-infected individuals. All PBL were stimulated with PHA (5µg/ml) for three days. Uninfected PBL were treated with IL-2 (10%) and/or HA.90 (25µg/ml) on day 3; mRNA levels were assayed on day 6 (A). Uninfected PBL were treated on day 3 with HA.90 (5µg/ml); on day 6, IL-2Rα protein levels were measured by flow cytometry analysis (B). PBL from two HIV-negative individuals (C and D) were treated on day 3 with IL-2 (10%) and/or HA.90 (25µg/ml), and assayed on days 6 (a panels) and 9 (b panels), for IL-Rα mRNA levels. PBL from two HIV-infected individuals (E and F) were treated on day 3 with IL-2 (10%) and/or HA.90 (25µg/ml) and assayed on day 6 for IL-Rα and IL-Rβ mRNA levels. Levels of mRNA were determined by Northern blot analysis using probes for human IL-2Rα and/or IL-2Rβ as indicated.

### C. Anti-HIV Activity of AVC

AVC has a marked anti-HIV effect as it inhibits virus replication in, and infectivity of, human lymphocytes. It also blocks the formation of syncytia between infected and noninfected lymphocytes.

Inhibition of viral infectivity in vitro was assessed according to standard procedures: levels of p24, an HIV core protein, and reverse transcriptase (RT) were assayed. As described below, AVC inhibited viral infectivity in Jurkat cells, peripheral blood lymphocytes (PBL) infected with HIV in vitro, PBL from HIV-infected individuals, and PBL infected during co-culture with PBL from HIV-infected individuals. Viral inhibition by AVC was achieved without significant cytotoxicity.

Peripheral blood lymphocytes (PBL) from a healthy donor were stimulated in vitro with phytohemagglutinin (PHA) (1 µg/ml) for 24 hours. 10⁷ cells were then inoculated with 1 ml of various dilutions (10⁻¹-10⁻⁴) of the virus, strain HB2-HIV, previously titrated and stored at -70°C. Incubation with the virus was done for one hour. The cells were then washed with phosphate buffered saline (PBS) in order to remove the viral inoculum and cultured in RPMI-1640 supplemented with 4 mM glutamine, 0.1 mM nonessential amino acids, 1 mM sodium pyruvate, 10 mM Hepes and 10 % fetal calf serum (FCS) in a 5% CO₂ atmosphere. Cultures were either treated with AVC or incubated in medium as a control. Culture supernatants were collected on day 5, 12, 19 and 26 and tested for the content (pg/ml) of p24 antigen using a commercial ELISA capture assay (Abbott Laboratories). The viability of the cell culture was checked by visual microscopic inspection. Parallel cultures were used to assess viability by Trypan blue exclusion. See Table I.

As indicated in Table II, AVC is effective at a concentration of 100 µg/ml and markedly reduced the production of p24 antigen. When used at 200 µg/ml its effect was more pronounced. If one considers the amount of virus produced on day 12 in the presence of 100 µg/ml of AVC, it is evident that this is similar to the amount of p24 produced in control cultures with 10⁻³ fold lower virus inoculum.

This result was confirmed in experiments with Jurkat cells, a human T lymphoma cell line, transfected with plasmid pNL4-3. This is a pUC18 plasmid vector that is a full length replication-and infection-competent chimeric DNA derived from NY5 (5') and LAV (3') genomic DNA. Upon transfection, Jurkat cells produced p24 for a period of up to three weeks. Two days before the beginning of the experiment the transfected cells were mixed with untransfected cells at a ratio of 1 to 1 in the presence or absence of AVC, and the mixture cultured in RPMI-1640 supplemented with 4 mM glutamine, 0.1 mM nonessential amino acids, 1 mM sodium pyruvate, 10 mM HEPES and 15 % FCS in a 5% CO₂ atmosphere. Culture supernatants were collected on day 2, 5 and 8, and tested for the content (pg/ml) of p24 antigen using a commercial ELISA capture assay (Abbott Laboratories). Total cell count was determined by Coulter counter and cell viability was checked by Trypan blue exclusion. The results of this experiment are summarized in Figures 1, 2 and 3.

The production of p24 antigen on day 8, the end-point of the experiment, was markedly reduced over control cultures (Figure 1). This effect was obtained with a concentration of AVC between 100 and 500 µg/mi. On day 2 all cultures treated with AVC produced higher amounts of p24. This can be interpreted to indicate that AVC has a short term stimulatory effect on T cells and that this is mirrored by a transient phase of virus replication. When the variation percent in p24 production in cells treated with AVC versus untreated cells were measured it appeared that AVC reduced virus production progressively until p24 was barely detectable (Figure 2).

When HIV infectivity was assessed by the production of RT, similar results were obtained. Two days before the onset of the experiment, equal volumes of pNL4-3 transfected Jurkat cells were mixed with untransfected cells, and the mixture cultured in RPMI-1640 supplemented with 2 mM L-glutamine, 15% fetal bovine serum, and 1% Pen-strep (100 U Penicillin, 100 µg of Streptomycin/ml). The cells were subsequently incubated with 10µg/ml, 25µg/ml, 50µg/ml, 100µg/ml, and 200µg/ml AVC. Six days later, RT activity was determined by an established procedure in which 5µl of 30-fold concentrated cell culture medium samples were tested in the presence of Mg+ + for the production of DNA (as measured by the incorporation of ³H from synthetic template-primer sets Oligo dtrA and dtdA. For the former set, the template is RNA; for the latter it is DNA. DNA-polymerase (DNAP) was used as a control for cellular enzyme, purified HIV-1 RT was used as a control for viral enzyme, and supernatant from infected untreated cells was used as a positive control. As shown in Figure 4, AVC concentrations from 10 to 200µg/ml dramatically reduced reverse transcriptase production. Maximal inhibition was observed after treatment with 50µg/ml AVC.

Inhibition of HIV infection by AVC was also demonstrated in PBL from HIV-infected individuals as well as PBL infected during co-culture with PBL from HIV-infected individuals. PBL were obtained by separation of freshly drawn blood on Ficoll-Hypaque (Histopaque 1077, Sigma) gradients and stimulated with 5µg/ml phytohemagglutinin (PHA, Burroughs-Wellcome) for 48 hours. Subsequently, fresh RPMI-1640 medium with 15% fetal calf serum (FCS), 2mM glutamine, and 10% interleukin-2 (IL-2, Boehringer-Mannheim) was supplied. PBL from HIV-1 positive donors were treated with 10, 25, 50, 100, and 200 µg/ml AVC. RT levels were assessed on day 6 by the method described above. Production of p24 was assayed on days 6, 9, 12, and 15 using HIVAG-1 ELISA test kits (Abbott Laboratories). The cut off (CO) value for ELISA kit was less than 0.1 (0.093) OD units. For both assays, untreated infected cells (C(I)) served as the positive control and uninfected cells (C(UN)) served as the negative control.

Co-culturing of PBL from HIV-infected individual and uninfected PBL resulted in the transmission of HIV-1 to the previously uninfected PBL. After three days of co-culture, PBL were treated with 10, 25, 50, 100, and 200 µg/ml) AVC. RT activity was assayed on day 6. Untreated infected cells (Cl) served as the control. Co-cultured cells treated with 10% IL-2 (A) or 25µg/ml AVC (B) on day 3 were assayed on days 6, 9, 12, and 15 for p24 levels. The negative control and cut off value for the ELISA kit are labeled C and D, respectively. See Figure 5.

Figure 5 demonstrates that AVC concentrations from 10-200µg/ml significantly inhibited the production of RT and p24 in PBL obtained from HIV-infected individuals and in PBL infected during co-culture with said cells. For PBL from HIV-infected individuals, all tested concentrations of AVC were similarly effective. For co-cultured PBL, maximal inhibition resulted from treatment with 100 or 200µg/ml.

The total number of cells during the experiment were also monitored. As shown in Figure 3 after 8 day culture with various amounts of AVC the total number of viable cells did not vary from that of control cultures incubated in medium alone. This was true both in the case of uninfected and infected cells. After 8 days incubation in the presence of AVC the percent variation of total number of viable cells over the total number of viable cells in the untreated cultures was within a 15% range, suggesting that cells tolerate well the treatment with AVC and no cell death nor cytopathology occurred. The viability was also comparable between treated and untreated groups with the single exception of the group treated with 500 µg/ml of AVC. Therefore, one can conclude that AVC is not toxic for the cells at a concentration of 200 µg/ml.

Additional experiments investigated the effects of AVC treatment on cell viability and proliferation of uninfected PBL and PBL from HIV-infected individuals. Freshly obtained PBL were isolated from heparinized blood using a Ficoll-Hypaque density gradient, washed, and resuspended in RPMI-1640 containing 15% FCS and 5 µg/ml purified PHA. On day 3 medium supplemented with 10% IL-2 was supplied and 0, 3, 6, 12, and 25µg/ml AVC was added. On day 6, viable cell numbers were determined by Trypan blue dye exclusion test and were confirmed with Coulter particle counter. The data shown in Figure 6 represent the average of two individuals in each case. In a slightly different experiment, shown in the Figure 7 inset, total cell number of untreated PBL and PBL treated with 25µg/ml AVC on day 3 were compared on day 4. PBL from five HIV-infected individuals were tested separately.

A longitudinal study of the effect of AVC on proliferation of PBL from HIV-infected individuals was also conducted. PBL were cultured for the first three days as described above. On day 3, PBL were treated with 10% IL-2 or 10% IL-2 plus 25µg/ml AVC. On day 6, IL-2 was given to some of the cells previously treated with AVC alone and 10% IL-2 was given to some of the previously untreated cells. Viable cell number was assessed every three days from day 3 to day 12. Untreated PBL served as a control. Data shown in Figure 7 represent average values of three donors tested separately. All HIV-1 infected donors were under AZT treatment.

Figures 6 and 7 demonstrate that more viable cells were present in cultures treated with AVC than in untreated cultures. This indicates that AVC preserves viability of PBL from HIV-infected individuals.

Liquid chromatographic (LC) fractionation of AVC was carried out by gel permeation chromatography (GPC). Five to ten gm of AVC was dissolved in 100 ml. of 0.2 M NaNO₃ solution in water. Solutions were filtered through 0.2 µm Anotop Plus inorganic filters (Altech Associates, Deerfield, IL). Injections were then made from a Theodyne Model 7125 valve (Cotati, CA) via a 10 mm³ loop onto the GPC column, an Ultrahydrogel Linear System (Waters Associates, Milford, MA) 300 X 7.8 mm internal diameter, containing particles of 6-13 µm. The exclusion range of the column extended from 1X10³ to 7X10⁶ Da (polyethelene oxide, polyacrylic acid standards; Phenomenex, Torrance, CA). The Hewlett-Packard (Avondale, PA) liquid chromatograph was comprised of a model HP 1050 dual pump system, an HP 1047A refractive index detector set to 35°C and 2⁹ attenuation, and an HP 3396-II integrator/plotter. The mobile phase, 0.2M Nano₃, was maintained at a flow rate of 0.5 cm³/min. W.W. Yau and D.D. Bly, *ACS Symposium Series 138*, 197-206(1980); W.W. Yau, J. J. Kirkland, D.D. Bly, *Modern Size-Exclusion Liquid Chromatography,* Wiley, New York, (1979).

AVC GPC fractions 1 to 5 were tested at varying concentrations for their effects on cell viability and production of p24 and reverse transcriptase in PBL infected in vitro with HIV-1₄₁₀₅. Cell viability and p24 expression were assessed after six days of treatment with different AVC fractions diluted 1:5, 1:10, 1:100, and 1:500 (original concentration = 5µg/ml). Untreated cells, buffer-treated cells, infected cells (C(I)) and uninfected cells (C(UN)) served as controls. Reverse transcriptase was assayed after 6 and 9 days of treatment with 1:10 dilutions of different AVC fractions. Controls included infected cells (C(I)), uninfected cells (C(UN)). RT, and DNAP. One day prior to supernatant collection for p24 or RT analysis, cell number was adjusted to approximately 1x10⁶/ml.

Figure 8 demonstrates that 1:10 dilutions of AVC fractions 2, 3, and 4 virtually eliminated p24 production. No reduction in cell number relative to untreated infected cells was seen in cells treated with a 1:10 dilution of AVC fractions 2 or 3; cell number was reduced by half in cells treated with a 1:10 dilution of AVC fraction 4. Thus, AVC fractions 2, 3, and 4 mimic the effects of unfractionated AVC.

In a second attempt to purify the active moiety from crude humic acid AVC reverse phase liquid chromatography was used. Sample solutions of 5-10g/cm³ AVC were prepared in 0.05-0.2M aqueous NaNO₃ and either decanted or filtered through 0.2µm Anotop Plus (Alltech Associates) inorganic membrane filters. The sample was then injected from a Rheodyne Model 7125 valve via a 1cm³ loop into a 150mm x 10mm RPLC column packed with 10µm particles. The RPLC column was attached to a Hewlett-Packard Model 1050 liquid chromatograph consisting of a dual pump system, a variable wavelength UV/Vis detector, and a Model HP 3396-II integrator/plotter. The column's mobile phase flow rate was maintained at 5cm³ per minute throughout the experiment. In the initial work, 5 peaks were collected (Figure 9) and studied.

Subsequently, a sixth peak was visualized that was eluted at the column with six peaks of fractionated material were observed (Figure 10): an initial "solvent" peak (15% w/v), three peaks comprising the main body of the material (10, 4, and 11 % w/v, respectively), a peak which trailed the fourth peak (9% w/v), and a final peak (51 % w/v) that eluted when the column was washed with methanol 10% 0.1N NaOH and 90% methanol. Individually collected eluates from a common peak were pooled and designated AVC fractions 1 to 6. Fractions 1 to 6 were tested for inhibition of p24 and cell viability (Figure 11). Fraction 4 is best with regard to inhibition of p24 and increased cell count combined.

In an effort to identify the molecular mechanism of AVC inhibition of HIV infectivity, AVC fractions 2, 3 and 4 were tested for ability to inhibit directly the activities of reverse transcriptase (RT) and DNA polymerase (DNAP) (Figure 12 and 13). The activities of RT and DNAP were assayed in vitro by the production of DNA from synthetic template-primer sets oligo dtrA and dtdA. Since the template for oligo dtrA is RNA, DNA production from dtrA is primarily due to RT activity. Similarly, DNA production from oligo dtdA, which has a DNA template, is primarily due to DNAP.

When Jurkat cells infected with PNLF-3 virus were tested with AVC fractions 1 to 5 multiply spliced with HIV-1 mRNA decreased dramatically with fraction 3 to 5 (Figure 12, upper panel). The effect of AVC fractions on unspliced HIV-1 mRNA was mild (Figure 12, lower panel). These observations suggest that AVC may influence post transcriptional events or inhibit secondary infection by HIV-1.

Treatment of infected Jurkat cells with AVC fraction 4 had effect on the pattern of proviral DNA (Figure 13). To follow the kinetics of HIV-1 DNA synthesis in cells treated with different fractions of AVC, we used primers amplifying "early", "intermediate" and "full length" DNA. Sakela *et al.,* J. Virol. 67, 7423 (1993); Saksela, *et al.,* Proc. Natl. Acad. Sci. 91, 1104 (1994). Zack, *et al.,* Cell 61, 213 (1990).

(DNA-PCR): Cells were washed in Hank's BSS and lysed in lysis buffer [10 mM Tris HCI (pH 8.0), 10mM EDTA (pH 8.0), and 0.7% SDS]. DNA was extracted by phenol-Chloroform procedure and finally precipitated with ethanol. DNA was dissolved in TE buffer (5µl/0.3X10⁵ cells). 5µl of DNA was used for PCR amplification in tightly fitting tubes in a Perkin-Elmer Cetus Gene Amp PCR system as follows: The reaction was carried out at 95°C for 15 sec. and 60°C for 1 min. for 33 cycles. Sequences for the primer sets used are given below:
(RT-PCR): mRNA was extracted using Quick Prep micro purification kit from Pharmacia. cDNA synthesis was done using 1st cDNA kit from Clontech. After RT reaction 20 µl of diluted (1:100) cDNA was added to 30 µl of PCR mixture. The reaction was carried out for 15 sec. at 95°C and 1 min. at 60 °C for 30 cycles (β-action = 25 cycles). PCR products were analyzed on 2% agarose gel. We found a small decrease in proviral DNA from cells treated with fractions 2 and 3 using primer set M667 and AA55. A decrease in specific PCR product was observed (Figure 13, upper panel). PCR analysis using a second set of primers (SK 38/39) also gave similar results (Figure 13, mid-panel) and a third set of primers (M667/M661) gave reduced DNA product (Figure 13, lower panel). Lack of specific signal in lane 4 could be attributed to the presence of inhibitors of DNA polymerase in this fraction. To answer this question diluted fraction 4 material was used in place of distilled water in PCR analysis. As shown in Figure 14, fraction 4 inhibited PCR amplification in a dose dependent manner.

Table III demonstrates that 10µl of AVC fraction 4 reduces DNAP activity to 2% of normal levels and lowers RT activity to 42% of normal levels.

AVC also effectively prevents the formation of syncytia between infected and uninfected cells. An assay that utilizes the CD4+ human T cells MOLT4 and the human T cell line 8E5 which harbors an integrated defective HIV-LAV retrovirus genome [Folks *et al.* J. Exp. Med. 164, 280 (1986)] was used. Briefly, 8E5 (1.2x10⁶/ml) and MOLT4 (2x10⁶/ml) cells were resuspended in complete RPMI medium, mixed vol:vol and 100 ml of the mixture incubated in 96-well plates (round bottom) (Costar) at 37 °C in a humidified CO₂ incubator with orbital shaking every 5'. After 30' the plates were spun for 5' at 800 rpm and incubated for 3 hours at 37°C. The cells were transferred to a 96-well plate (flat bottom) (Costar) and the formation of syncytia was determined by microscopic inspection on an inverted microscope. Syncytia were defined as cells having a balloon degeneration. Percent inhibition was calculated as follows: (No. syncytia with AVC - No. syncytia with medium alone/No. of syncytia with medium alone) X 100.

Figure 15 shows that AVC is completely effective in blocking the formation of syncytia when used at concentrations of 50 µg/ml. Lower concentrations (25 and 12.5 µg/ml.) are also highly effective (not shown). Therefore, in addition to blocking the production of HIV by infected cells AVC also blocks the fusion between infected and uninfected cells and the formation of syncytia.

### D. Costimulatory Effect of AVC on IL-2 Production

The long-term effects of AVC on cell viability and proliferation of PBMC activated with PHA plus IL-2 was studied. AVC did not significantly modify the proliferative response. However, when PBMC were activated with PHA and IL-2 was added on day 6, AVC enhanced the proliferative response relative to untreated cells given IL-2 on day 6. It is of note that cells incubated with AVC alone since day 3 underwent a proliferative phase which peaked on day 9. This was not seen in control cells incubated in medium alone or in cells given IL-2 on day 6.

When human PBLs are stimulated in vitro by plant lectins, e.g., phytohemagglutinin (PHA) or concanavalin A (Con A) in the presence of AVC, the amount of IL-2 produced is in most cases enhanced. Lectins are proteins of plant origin that bind specifically to complex carbohydrate groups.

Typically, peripheral blood lymphocytes (PBL) from normal donors are incubated (2 x 10⁶ cells/ml) in RPMI-1640 medium supplemented with 4 mM glutamine, 0.1 mM nonessential amino acids, 1 mM sodium pyruvate, 10 mM HEPES and 10 % FCS and containing either Con A (5 µg/ml) or PHA (10 µg/ml) as the mitogen. AVC was added at the initiation of the culture at the dose of 12 µg/ml. The cultures were then incubated in a 5% CO₂ atmosphere. Culture supernatants were collected on day 2, 3 and 4 and tested for the content (Units/ml) of IL-2 using a biological assay consisting in measuring the proliferative response of murine CTLL-2 T cells whose growth in culture is strictly dependent on the presence of IL-2 in the medium. The proliferative response (³H-Thymidine incorporation) of CTLL-2 cells was plotted on a reference curve constructed with a standard amount of IL-2 and the experimental values calculated accordingly.

The effect of AVC on cytokine production by normal PBMC activated with Con A or PHA was examined. Cytokine production was monitored using as an indicator the IL-2 dependent CTLL-2 murine cell line. During Con A activation AVC markedly augmented cytokine production in 12 out of 17 cases (70%) with a mean stimulation index of 2.2 (Figure 16). The same phenomenon was observed when PBMC were stimulated with PHA (Figure 16).

The effect on IL-2 production by AVC was not due to a direct proliferative effect on PBL in culture (Table IV) nor did it have a direct effect on IL-2 production by Jurkat or LBRM-33 T lymphoma cells (Table V).

In addition to stimulating IL-2 production, AVC inhibits the expression of the IL-2 receptor. The receptor for IL-2 (IL-2R) is composed of at least two distinct subunits, IL-2Rα (a 55 kDa protein) and IL-2Rβ (a 75 kDa protein). As shown in Figure 17, Northern analysis revealed reduced expression of IL-2Rα and IL-2Rβ mRNA in normal PBL treated for 24 hours with PHA plus 25µg/ml AVC relative to cells stimulated with PHA alone or PHA plus IL-2. This effect was mirrored by a diminished surface expression of IL-2Rα protein, as measured by flow cytometry, in PBL treated with 25µg/ml AVC. Similarly, IL-2Rα mRNA could not be detected on day 6 in PBL activated by PHA for three days and treated with 25µg/ml AVC on day 3. However, down-regulation was transient -- on day 9, levels of IL-2Rα mRNA expression were comparable to untreated cells. The same analysis was done with cells from two HIV-1 infected individuals. In one case, the levels of IL-2Rα and IL-2Rβ mRNA were lower in cells receiving IL-2, AVC, or IL-2 plus AVC than in untreated cells; in the other case, IL-Rα mRNA was undetectable in AVC-treated cells but detectable in IL-2-treated and untreated cells while IL-Rβ was undetectable in all cells, including those not treated.

The concomitant increase in IL-2 production and decrease in expression of the IL-2 receptor seen in AVC treated cells may partially explain AVC's ability to protect the viability of PBL from HIV-infected individuals. The rate of IL-2 mediated cell division is known to depend upon IL-2 concentration, IL-2R density and the duration of IL-2/IL-2R interaction. Perhaps the cell cycle becomes desynchronized as a result of AVC treatment. HIV-infected PBL with a protracted time for IL-2 utilization, DNA replication and cell division may retain viability longer.

### E. Collective Data

**TABLE I**

| **Total Number of Cells in Cultures Containing AVC** | | |
|---|---|---|
| AVC | Number of Cells | |
| [µg/ml] | Noninfected | Infected |
| 0 | 1.46 X 10⁶/ml | 3.44 X 10⁶/ml |
| 100 | 1.68 X 10⁶/ml | 4.1 X 10⁶/ml |
| 200 | 1.58 X 10⁶/ml | 4.6 X 10⁶/ml |
| 500 | 3.42 X 10⁶/ml | < 10⁴/ml |

**TABLE II**

| AVC Inhibits the Infectivity of Human PBL by HIV | | | | | |
|---|---|---|---|---|---|
| | | p24 antigen (pg/ml) | | | |
| AVC | Virus Dilution | Day 5 | Day 12 | Day 19 | Day 26 |
| 100µg/ml | 10⁻¹ | 510 | 250 | 27,800 | Term |
| | 10⁻² | 34 | 245 | 3,900 | Term |
| | 10⁻³ | 0 | 18 | 126 | Term |
| | 10⁻⁴ | 0 | 10 | 208 | Term |
| 200µg/ml | 10⁻¹ | 225 | 400 | 1,740 | Term |
| | 10⁻² | 14 | 23 | 28 | 52 |
| | 10⁻³ | 0 | 0 | 20 | 28 |
| | 10⁻⁴ | 0 | 0 | 0 | 0 |
| 0 | 10⁻¹ | 9,500 | 101,000 | Term | |
| | 10⁻² | 770 | 22,500 | Term | |
| | 10⁻³ | 111 | 1,440 | Term | |
| | 10⁻⁴ | 0 | 37 | 234 | Term |

**TABLE III**

| **RT Result** | | | | |
|---|---|---|---|---|
| No. | Sample | dtra Mg + + | No. | dtda Mg + + |
| 1 | Blank | 512 | 15 | 993 |
| 2 | RT | 40,119 | 16 | 13,286 |
| 3 | DNA Polymerase | 17,402 | 17 | 162,707 |
| 4 | RT/f₂ 10λ | 28,700 | 18 | 4,339 |
| 5 | RT/f₃ 10λ | 23,420 | 19 | 4,854 |
| 6 | RT/f₄ 1λ | 44,293 | 20 | 5,936 |
| 7 | RT/f₄ 5λ | 19,363 | 21 | 4,710 |
| 8 | RT/f₄ 10λ | 16,875 | 22 | 3,785 |
| 9 | DNAP/f₂ 10λ | 14.070 | 23 | 18,611 |
| 10 | DNAP/f₃ 10λ | 1,980 | 24 | 1,791 |
| 11 | DNAP/f₄ 1λ | 30,408 | 25 | 49,304 |
| 12 | DNAP/f₄ 5λ | 1,229 | 26 | 4,718 |
| 13 | DNAP/f₄ 10λ | 641 | 27 | 4,066 |
| 14 | PNL 43/J | 4,543 | 28 | 1,909 |

**TABLE IV**

| **AVC Alone Does Not Induce Proliferation of Human PBL** | | | | |
|---|---|---|---|---|
| Experiment | Cells Treatment | 24 Hours | 48 Hours | 72 Hours |
| 1 | Con A | 3,772 | 37,839 | 42,357 |
| | AVC | 360 | 701 | 1,540 |
| 2 | Con A | 182 | 23,767 | ND |
| | AVC | 293 | 42,285 | ND |

**TABLE V**

| **AVC Does Not Modify the Production of IL-2 by T Lymphoma Cells** | | | | |
|---|---|---|---|---|
| Time (Hours) | Jurkat Cells | | LBRM-33 Cells | |
| | Con A | Con A + AVC | Con A | Con A + AVC |
| 24 | 3,815 | 5,484 | 28,774 | 29,211 |
| 48 | 10,061 | 4,038 | 28,880 | 27,927 |

### F. Chemical Analysis

In view of the consideration that hydroxyquinones are considered important structural groups of the organic molecules constituting humic acid, a synthetic approach was undertaken in order to better determine the chemical structures of the compounds contained in the active fractions that were identified as per the above data. To this end a number of synthetic compounds were prepared, namely, 2-5-dihydroxybenzoic acid (compound O), 3-4-dihydroxyphenylacetic acid (compound 2), 3-4-dihydroxymandelic acid, otherwise known as alpha,3,4-trihydroxyphenylacetic acid, (compound 3) and 2,5-dihydroxyphenylacetic acid (compound 4). Each of these compounds were subjected to reverse phase liquid chromatography under conditions identical to the reverse phase liquid chromatography done with the crude humic acid as discussed above and as is depicted in Figure 10 hereof. Judging from the peak areas of the resultant chromatograms, it was determined that compound 4 gave a polycondensate that corresponds to about 90% pure "fraction 4" of the crude humic acid as set forth above.

Details concerning the synthesis, isolation, analysis and results of the foregoing conclusions are as follows:

### Synthesis

Dissolve 10 mmol compound in 300 cm³ 0.1 M NaOH. Adjust the solution pH to 8.5 with 6M HCI. Add 2.5 mmol sodium periodate (NalO₄; 0.5 µg). Warm to 50°C for 30 min., then allow solution to stand overnight at room temperature.

### Isolation

Make the solution pH to less than 1 with 6M HCI. Allow the solution to stand overnight. Decant the mother liquor and discard. Save the precipitate. (Centrifuge the acid solution, if necessary, to isolate the solid precipitate at 300 rpm for 10 min.) Transfer the precipitate to 40 cm³ freeze-drying flasks with 1M HCI. Bring the volume of precipitate and solution to ca. 25 cm³ with 1M HCI. Centrifuge at 3000 rpm for 10 min. Discard the mother liquor and add a fresh portion, ca. 25 cm³ of 1M Hcl to each precipitate. Repeat the centrifugation. Discard the mother liquor, and freeze dry the precipitate.

### Analysis

Reverse-phase liquid chromatography (RPLC) is used for the analysis. The instrument employed in the present instance was a Hewlett-Packard Model 1050, equipped with a variable-wavelength UV/Vis detector. The detection wavelength is 340 nm. The RPLC column is a Hamilton PRP-1 unit, 4.1 X 150 mm, containing 10µ particles. A special universal buffer mobile phase is used for the analysis. Solvent A is made from 0.20M boric acid, 0.20M phosphoric acid and 0.20M acetic acid. Thus, 250 cm³ of each acid solution is mixed together, and then made up to 1L with 0.05M sodium nitrate. Solvent B is 0.50M sodium hydroxide, while Solvent C is simply pure methanol. The mobile-phase solvent gradient program is as follows: Solvent A - 60%, plus Solvent B - 40% at the start. The composition is brought to 100% B over the course of 15 min., then to 10% B and 90% C over the next 5 min. The latter mobile-phase composition is then held constant for 10 min. The sample loop size is 6 mm³. The crude HA sample is prepared by dissolving 5g material in 100 cm³ water, pH ca. 8-10. The solution is allowed to stand overnight, and is then centrifuged at 3000 rpm for 10 min. Synthetic materials appear to dissolve without difficulty in weak base.

### Results and Discussion

The polycondensate of Compound 4 gives an RPLC trace that appears (by peak areas) to correspond to ca. 90% pure fraction 4 (see Figures 9 and 10).

### Concluding Remarks

In the in vivo toxicity study Balb C see mice of 10 to 12 weeks of age are injected intravenously with 0.5 ml of an amount of fraction 4 of the crude material separated by RPLC. Mice are injected for 7 consecutive days. At the end of the experiment blood and urine samples are collected for chemical analysis. Mice are sacrificed and autopsy performed with histopathology of various organs including liver, kidney, lung, and spleen. On the basis of this experiment LD-50 is established, as well as an assessment of the side effects of overdose of this material in vivo.

This invention teaches the general properties of a non-toxic natural, commercially available material, humic acid, as an inhibitor of HIV replication and as a costimulatory agent of CD4 T cells in the production of IL-2.

From the demonstrated first property it can be concluded that humic acid and the family of compounds or other components that belong to the humic acid family, natural or synthetic, are useful as effective therapy for HIV infection and AIDS. This is supported by the demonstrated efficacy of humic acid as an anti-viral agent together with the lack of cytotoxicity at the effective dose range.

The invention also pertains to a general immunostimulatory effect by humic acid. Thus, humic acid serves as a costimulant of the production of IL-2 by human CD4 T cells. IL-2 is a cytokine indispensable to many functions of the immune system including activation and proliferation of CD4 T cells, activation and proliferation of CD8 T cells, and induction and growth of natural killer (NK) cells. It has been amply documented that lymphocytes activated by IL-2 are involved in the protective immune response to tumors and viruses. This indicates that IL-2 is a lymphokine with pleiotropic effects on the immune system. An increase in the basal level of endogenous IL-2 like the one sustained by humic acid may be also beneficial to AIDS patients. A benefit can be seen in the maintenance of CD4 cells and their function, the prevention of their death by apoptosis, a boost in the induction and function of CD8 T cells a fact that directly or indirectly could be of great advantage to infected individuals.

The present invention is based upon the aforementioned experiments which in effect tested commercial Humic Acid preparations in *in vitro* tests demonstrating its effects in anti-HIV activity and stimulatory effect on IL-2 production. Based upon these findings, it would be within the skill of the art to produce pharmaceutically acceptable preparations of such Humic Acid components by compounding with known and acceptable excipients. The Humic Acid would be administered in amounts sufficient to demonstrate anti-HIV activity and/or stimulation of IL-2 production *in vivo* when administered via such pharmaceutically acceptable compositions. Based upon the data described herein, one would administer the Humic Acid preparations hereof to an individual at a dose of from about 500 mg. to about 1 g./person.

Thus, considering that a dose of 100-200 µg/ml. (level concentration) is efficacious and nontoxic and that the blood mass is ∼ 5000 ml. where biodistribution is stable, an efficacious dose, preferably by intravenous administration would be at least about 500 mg. per person (7.5 mg/kg body weight).

The preparations hereof would be administered in a manner thought to most efficient for bringing rapid contact of the active component with the PBL of an individual patient. Preferably, the administration would be intravenous. However, other routes of administration, e.g., oral, intravaginal and rectal can not be excluded. Assuming good absorption, an intramuscular injection of 1 g (15 mg/kg body weight) will give reasonably good peaks at 3 and 5 hours.

## Claims

1. Use of the humic acid for the preparation of a medicament for the inhibition of human immunodeficiency virus in leukocytes infected by said virus.

2. The use of claim 1, wherein said medicament is used to inhibit human immunodeficiency virus infection by contacting leukocytes of an individual infected with said virus *ex vivo.*

3. The use of claim 1, wherein said medicament is used to inhibit human immunodeficiency virus infection by contacting leukocytes of an individual infected with said virus, said leukocytes comprising peripheral blood mononuclear cells, lymphocytes resident in lymphoid organ and lymphocytes infiltrating tissues other than lymphoid organ.

4. Use of the humic acid as a co-stimulant for the preparation of a medicament for immunostimulating IL-2.

5. Use of humic acid as an adjuvant for the preparation of a medicament for the immunovaccination of a patient against human immunodeficiency virus or acquired immunodeficiency syndrome, or depressed immune function.

## Patentansprüche

1. Verwendung von Huminsäure zur Herstellung eines Medikaments zur Hemmung von Human-lmmunschwäche-Virus in Leukozyten, die mit diesem Virus infiziert sind.

2. Verwendung nach Anspruch 1, worin das Medikament verwendet wird, um Human-lmmunschwäche-Virus-Infektion zu hemmen, indem Leukozyten eines mit dem Virus infizierten Individuums ex vivo kontaktiert werden.

3. Verwendung nach Anspruch 1, worin das Medikament verwendet wird, um Human-Immunschwäche-Virus-Infektion zu hemmen, indem Leukozyten eines mit dem Virus infizierten Individuums kontaktiert werden, wobei die Leukozyten mononukleare periphere Blutzellen, in Lymphorgan vorliegende Lymophozyten und Lymphozyten umfassen, die andere Gewebe als das Lymphorgan infiltrieren.

4. Verwendung von Huminsäure als Co-Stimulans zur Herstellung eines Medikaments zur Immunstimulierung von IL-2.

5. Verwendung von Huminsäure als Adjuvans zur Herstellung eines Medikaments zur Immunimpfung eines Patienten gegen Human-lmmunschwäche-Virus oder erworbenes Immunschwächesyndrom oder herabgesetzte Immunfunktion.

## Revendications

1. Utilisation de l'acide humique pour la préparation d'un médicament pour l'inhibition du virus de l'immunodéficience humaine dans des leucocytes infectés par ledit virus.

2. Utilisation de la revendication 1, où ledit médicament est utilisé pour inhiber une infection par le virus de l'immunodéficience humaine par mise en contact des leucocytes d'un individu infecté par ledit virus *ex vivo.*

3. Utilisation de la revendication 1, où ledit médicament est utilisé pour inhiber l'infection par le virus de l'immunodéficience humaine par mise en contact des leucocytes d'un individu infecté par ledit virus, lesdits leucocytes comprenant des cellules mononucléaires de sang périphérique, des lymphocytes résidant dans l'organe lymphoïde et des lymphocytes infiltrant les tissus autres que l'organe lymphoïde.

4. Utilisation de l'acide humique comme co-stimulant pour la préparation d'un médicament pour immunostimuler IL-2.

5. Utilisation de l'acide humique comme adjuvant pour la préparation d'un médicament pour l'immunovaccination d'un patient contre le virus de l'immunodéficience humaine ou le syndrome d'immunodéficience aquise, ou une fonction immune abaissée.
